(19) Europäisches Patentamt
European Patent Office
Office européen des brevets

(11) **EP 3 468 499 B1**

(12) **EUROPÄISCHE PATENTSCHRIFT**

(45) Veröffentlichungstag und Bekanntmachung des Hinweises auf die Patenterteilung:
**24.06.2020 Patentblatt 2020/26**

(21) Anmeldenummer: **18728603.4**

(22) Anmeldetag: **29.05.2018**

(51) Int Cl.:
**A61B 50/30** *(2016.01)*      **A61B 17/86** *(2006.01)*

(86) Internationale Anmeldenummer:
**PCT/EP2018/064104**

(87) Internationale Veröffentlichungsnummer:
**WO 2019/042603 (07.03.2019 Gazette 2019/10)**

(54) **BOHRDRAHTHÜLSE**

DRILL WIRE SLEEVE

GAINE DE FORET FILIFORME

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.08.2017 DE 102017215142**

(43) Veröffentlichungstag der Anmeldung:
**17.04.2019 Patentblatt 2019/16**

(73) Patentinhaber: **Aesculap AG**
**78532 Tuttlingen (DE)**

(72) Erfinder:
• **WEISSER, Daniel**
**78048 Villingen-Schwenningen (DE)**
• **MAUTHE, Fabian**
**78607 Talheim (DE)**

(74) Vertreter: **Koller, Tobias Kilian**
**Aesculap AG**
**Abteilung Recht, Patente und M&A**
**Am Aesculap-Platz**
**78532 Tuttlingen (DE)**

(56) Entgegenhaltungen:
**DE-A1- 3 119 173     US-A1- 2009 266 890**

• **Interlockmed: "Catalogue 2016", A/V A to Z: An Encyclopedic Dictionary of Media, Entertainment and Other Audiovisual Terms, 21. April 2016 (2016-04-21), XP055501724, ISBN: 978-0-7864-9556-6 Gefunden im Internet: URL:https://www.interlockmed.com/media/wysiwyg/pdf/katalog/ZSVA/CSSD_-_Catalogue_2016_low.pdf [gefunden am 2018-08-23]**

EP 3 468 499 B1

**Beschreibung**

Die vorliegende Erfindung betrifft eine Bohrdrahthülse, also eine Lagerungshülse für Bohrdrähte, auch Kirschnerdrähte oder K-Drähte genannt, welche eine Sterilisierung der Bohrdrähte ohne Entnahme derselben aus der Lagerungshülse ermöglicht, welche die Bohrdrähte vor Beschädigung schützt und welche es dem Nutzer ermöglicht, Bohrdrähte komfortabel in die Lagerungshülse einzuführen. Vorzugsweise können die K-Drähte in der Bohrdrahthülse vor der Sterilisation auch gereinigt werden.

Bohrdrähte werden zum Beispiel verwendet, um Knochensegmente bei einem Bruch zueinander zu positionieren und zu fixieren. Die Bohrdrähte werden vor allem bei perkutanen Operationen als Führungselemente für kanülierte Pedikel-Schrauben verwendet. Hierbei wird der Draht durch einen Trokar in das betroffene Knochenstück eingeschraubt bzw. eingesteckt, wobei die gewünschte Tiefe über eine auf den Draht aufgebrachte Skala bestimmt werden kann. Durch diese Skala wird auch die richtige Schraubenlänge bestimmt. Über den so befestigten Bohrdraht wird nun eine kanülierte Schraube aufgezogen und in der vorgegebenen Richtung in das Pedikel geschraubt. Anschließend wird der Draht wieder entfernt. Ein Bohrdraht ist ein länglicher, drahtförmiger Körper mit einer distalen Bohrdrahtspitze und einem proximalen Ende. Die Bohrdrahtspitze muss dabei nicht unbedingt spitz ausgebildet sein, sondern kann auch z.B. stumpf ausgebildet sein und mit einer gewissen Oberflächenrauheit versehen sein, sodass mit solch einer Bohrdrahtspitze genau genommen nicht gebohrt wird, sondern mehr geschliffen. Es kann aber auch eine echte Bohrspitze an der Bohrdrahtspitze vorgesehen sein. Auch gibt es Bohrdrähte mit einer spitz zulaufenden Bohrdrahtspitze, sogenannte Trokar-Spitzen, welche auch noch mit einer Längsrändel oder Gewindeabschnitten versehen sein können. Es gibt auch Bohrdrahtspitzen mit seitlichen Abflachungen, mit Ösen in der Bohrdrahtspitze oder Bohrdrahtspitzen, die einfach rund enden. Ein Bohrdraht hat üblicherweise einen Durchmesser von ca. 0,6 mm bis 3,0 mm. Das proximale Ende des Bohrdrahts ist üblicherweise stumpf ausgebildet. Ein Bohrdraht kann entlang seiner Längsausdehnung Markierungen aufweisen, die dem Operateur intraoperativ anzeigen, wie tief der Bohrdraht bereits in das Gewebe eingedrungen ist. Für diese Anmeldung ist lediglich relevant, dass ein Bohrdraht einen länglichen Körper hat und ein distales Ende (die Bohrdrahtspitze) und ein proximales Ende aufweist.

Im Stand der Technik gibt es verschiedene bekannte Lagerungsarten für Bohrdrähte. Einerseits gibt es eine Art Sortierkasten für Bohrdrähte, in welchem für jede Gruppe von Bohrdrähten (z.B. für verschiedene Durchmesser oder verschieden ausgebildete Spitzen) ein einzelnes Fach vorgesehen ist, in welches die entsprechenden Bohrdrähte eingelegt werden. Der komplette Sortierkasten muss dann aber für eine Reinigung und zur Aufbewahrung in einen eigenen Siebkorb eingesetzt werden. Diese Art von Bohrdrahtlagerung hat den Vorteil, dass die Bohrdrähte leicht gereinigt werden können und gut aus der Lagerung entnehmbar sind. Allerdings hat diese Lagerungsart den Nachteil, dass die Bohrdrähte nicht gut geschützt sind, z.B. gegen eine Beschädigung, wenn die Lagerung aus dem Siebkorb entnommen ist. Außerdem benötigt diese Art der Bohrdrahtlagerung viel Platz und dieser ist insbesondere im OP sehr begrenzt. Die Firma MK Medical vertreibt solche Lagerungen auf ihrer Website mk-medical.com. Es gibt auch derartige Lagerungen, bei denen Lagerung und Siebkorb miteinander verbunden sind. Eine solche Lagerung ist beispielsweise in der Offenlegung CN 205359661 U gezeigt.

Die Firma Interlock Medizintechnik GmbH veröffentlichte (https://www.interlockmed.com/media/ wysiwyg/pdf/katalog/ZSVA/CSSD_-_Catalogue_2016_low.pdf, siehe Seite 88 und 89) eine Bohrdrahthülse gemäß dem Oberbegriff des Anspruchs 1.

In der Veröffentlichung US 2017/0065794 A1 ist eine andere Art einer Bohrdrahtlagerung gezeigt. Diese Art von Lagerung ist für besonders lange Bohrdrähte geeignet und hat einen im Wesentlichen schneckenförmigen bzw. schraubenförmigen, geschlossenen Aufnahmeraum aus Kunststoff, um einen Bohrdraht mit mehreren Wicklungen in sich aufzunehmen, wobei die

einzelnen Wicklungen voneinander durch Trennwände separiert sind. Eine solche Bohrdrahtlagerung eignet sich aber nicht für die Reinigung und Sterilisation eines Bohrdrahts.

Noch eine andere Art von Bohrdrahtlagerung ist die sogenannte Bohrdrahthülse. Bei der Bohrdrahthülse ist ein Hohlzylinder einseitig verschlossen und einseitig verschließbar, wobei die Mantelfläche des Hohlzylinders mit Durchbrechungen versehen ist, sodass Reinigungsfluid und Sterilisationsmedium in die Bohrdrahthülse eindringen können und die Bohrdrähte in der Bohrdrahthülse gereinigt und sterilisiert werden können. Solch eine Bohrdrahthülse kann einen unterteilten Innenraum aufweisen, sodass verschiedene Arten von Bohrdrähten in einer Bohrdrahthülse aufbewahrt werden können, ohne sich miteinander zu vermischen. Eine Vielzahl von Bohrdrahthülsen mit und ohne Unterteilung, mit rundem oder quadratischem Querschnitt, mit einer verschließbaren Endkappe oder zweien, mit Endkappen aus Kunststoff oder Metall usw. werden beispielsweise von der Firma Interlock auf ihrer Website interlockmed.com angeboten.

In der Offenlegungsschrift DE 31 19 173 A1 ist eine Bohrdrahthülse gezeigt, bei der ein im wesentlichen zylindrischer Körper an einer Stirnseite mit einem Schraubdeckel verschlossen ist und der Innenraum an der anderen Stirnseite trichterförmig zuläuft und eine zentrale Öffnung aufweist, welche geringfügig größer ist als der Querschnitt eines Bohrdrahts. Auf diese Weise wird eine Art Bohrdrahtspender erzeugt, der wie ein Zahnstocherspender funktioniert.

Die Mantelfläche des zylindrischen Körpers ist mit ovalen Durchbrechungen als Spülöffnungen versehen, welche regelmäßig in Längs- und Umfangsrichtung verteilt sind. Die Achsen der einzelnen ovalen Durchbrechungen sind in Längsrichtung und Tangentialrichtung der Hülse ausgerichtet.

Die bislang offenbarten Bohrdrahthülsen haben bei der Benutzung den Nachteil, dass das vorangehende Ende des Bohrdrahts beim Einführen des Bohrdrahts in die Bohrdrahthülse in eine der Durchbrechungen eindringen kann, welche als Spülöffnungen in der Mantelfläche der Bohrdrahthülse vorgesehen sind. Dann kommt das vorangehende Ende des Bohrdrahts, abhängig vom Einführwinkel, in Anlage mit der distalen Wandung der Durchbrechung. Hierbei ist es unerheblich, ob der Bohrdraht mit seiner Bohrspitze oder seinem proximalen Ende voran in die Bohrdrahthülse geschoben wird. Dieses Eindringen des vorangehenden Endes des Bohrdrahts erfolgt, da der Bohrdraht nie exakt gerade (also exakt parallel zu der Längsachse der Bohrdrahthülse) in die Bohrdrahthülse eingebracht wird. Der Effekt ist umso größer, je größer der Winkel $\alpha$ des Einbringens des Bohrdrahts in die Bohrdrahthülse gegenüber der Längsachse der Bohrdrahthülse ist. Ist der Winkel $\alpha$ relativ klein, gleitet das vorangehende Ende des Bohrdrahtes selbständig über die Kante, die die Wandung der ovalen Öffnung mit der Innenwandfläche des Aufnahmeraums der Bohrdrahthülse bildet. Ist der Winkel $\alpha$ sehr groß, kann es sogar sein, dass der Bohrdraht durch die Durchbrechung hindurch geschoben wird. In der Praxis ist dies aber eher unwahrscheinlich, da sich sehr große Winkel $\alpha$ durch den Nutzer leicht vermeiden lassen. In einem mittleren Bereich für den Winkel $\alpha$ kann es aber zu der vorstehend beschriebenen Anlage kommen. In solch einem Fall kann der Bohrdraht nicht weiter in die Bohrdrahthülse geschoben werden. Der Nutzer muss den Bohrdraht zunächst wieder ein Stück aus der Bohrdrahthülse heraus ziehen und den Bohrdraht unter einem kleineren Winkel $\alpha$ erneut hinein schieben. Dies ist für den Nutzer lästig und kostet Zeit. Im hektischen OP Umfeld kann dies Unruhe und Fehler nach sich ziehen. Auch eine Beschädigung des Bohrdrahts ist nicht ausgeschlossen. Beispielsweise kann der Bohrdraht beim erneuten Herausziehen auf den Boden fallen, von wo er aber nicht mehr aufgehoben werden kann, ohne dass die betreffende Person sich unsteril macht. Ein Aufheben eines auf den Boden gefallenen Bohrdrahts erfordert entweder eine Unterbrechung der OP um die betreffende Person erneut steril einzukleiden, oder der Bohrdraht bleibt bis zur Beendigung der OP am Boden Liegen und bildet eine stete Unfallquelle, da man leicht auf einem Bohrdraht ausrutschen kann.

Die Aufgabe der vorliegenden Erfindung besteht daher darin, eine Bohrdrahthülse zu schaffen, bei der ein Anschlagen des vorangehenden Endes eines Bohrdrahtes an einer distalen Wandung eines Reinigungslochs in einem üblichen Einführwinkel $\alpha$ eines Bohrdrahts in eine Bohrdrahthülse zuverlässig verhindert wird, wenn ein Bohrdraht in einem Winkel in eine Bohrdrahthülse eingebracht wird.

Die Aufgabe der vorliegenden Erfindung wird durch eine Bohrdrahthülse gemäß Anspruch 1 gelöst. Vorteilhafte Ausgestaltungen sind Gegenstand der abhängigen Ansprüche.

Eine Bohrdrahthülse gemäß der vorliegenden Erfindung weist eine rohrförmige, längliche Hülse mit einer Länge L, einem Innendurchmesser $d_i$ und einem Außendurchmesser $d_a$ auf. Darüber hinaus verfügt die Bohrdrahthülse über eine erste axiale Endkappe und eine zweite axiale Endkappe, wobei die erste axiale Endkappe und/oder die zweite axiale Endkappe mit einem Gewinde werkzeugfrei lösbar an der rohrförmigen, länglichen Hülse befestigbar ist. Eine solche werkzeugfrei lösbare Endkappe verschließt lösbar eine Einführöffnung der rohrförmigen, länglichen Hülse. Darüber hinaus weist die erste axiale Endkappe und/oder die zweite axiale Endkappe einen nicht rotationssymmetrischen Außenquerschnitt auf. Die rohrförmige, längliche Hülse verfügt in ihrer Mantelfläche über eine Vielzahl von ersten Durchbrechungen. Diese ersten Durchbrechungen sind länglich ausgebildet und in einem Winkel von 25° bis 65° gegenüber der der Längsachse der rohrförmigen, länglichen Hülse geneigt angeordnet.

Die gegenüber der Längsachse der Hülse geneigte Anordnung von länglichen ersten Durchbrechungen bewirkt einen besonderen Effekt. Wenn ein Bohrdraht in eine derart ausgebildete Bohrdrahthülse in einem mittleren Bereich eines Einführwinkels $\alpha$ eingeführt wird, kann es nach wie vor passieren, dass das vorangehende Ende in eine der ersten Durchbrechungen eindringt. In diesem Fall kommt das vorangehende Ende des Bohrdrahts auch in Kontakt mit der Innenkante der Durchbrechung, welche im Übergang von der Wandung der Durchbrechung mit der Innenwandfläche der Hülse gebildet ist. Da diese erste Durchbrechung nun aber länglich ausgebildet ist und zudem gegenüber der Längsachse der Hülse geneigt ist, ist diese Innenkante an der Stelle des Kontakts mit dem vorangehenden Ende des Bohrdrahts mit höchster Wahrscheinlichkeit ebenfalls geneigt (Ausnahme: der Kontakt findet exakt am distalen Ende dieser ersten Durchbrechung statt, was aber extrem unwahrscheinlich ist). Dann gleitet das vorangehende Ende des Bohrdrahts an dieser Kante entlang. Da diese Kante sich aber teilweise in Umfangsrichtung der Mantelfläche der Bohrdrahthülse erstreckt, verändert sich dadurch der Winkel $\beta$ zwischen dem vorangehenden Ende des Bohrdrahts und der Längsachse der Hülse, das heißt dieser Winkel $\beta$ wird kleiner, je weiter das vorangehende Ende des Bohrdrahts an der Innenkante der ersten Durchbrechung entlang gleitet. Dies geschieht entweder, indem der Bohrdraht elastisch verformt wird (d.h. $\beta < \alpha$), oder indem der Nutzer, der den aus der Bohrdrahthülse heraus ragenden Abschnitt des Bohrdrahts hält, dieser Bewegung folgt (d.h. $\beta = \alpha$), die von dem vorangehenden Ende des Bohrdrahts vorgegeben wird. Wenn der Winkel $\beta$ klein genug ist, dann gleitet das vorangehende Ende des Bohrdrahts plötzlich leicht über die Innenkante und der Bohrdraht kann ganz leicht weiter in die Bohrdrahthülse eingeschoben werden. Ein kurzzeitiges Herausziehen und neu Einführen des Bohrdrahts in die Bohrdrahthülse kann somit effizient verhindert werden und somit die vorstehend beschriebenen Probleme vermieden werden.

Die länglichen ersten Durchbrechungen sind bevorzugt regelmäßig entlang des Umfangs der länglichen Hülse und entlang ihrer axialen Erstreckung angeordnet. Beispielsweise sind jeweils 4 erste Durchbrechungen in Umfangsrichtung angeordnet und bilden so eine ringförmige Struktur. Diese Struktur wiederholt sich dann in axialer Richtung der länglichen Hülse mit einem gewissen Abstand zueinander, wobei die Struktur in Umfangsrichtung der Hülse auch um einen bestimmten Winkel zueinander versetzt angeordnet sein kann.

Vorzugsweise sind die länglichen ersten Durchbrechungen in einem Winkel von 28° bis 55° gegenüber der Längsachse der Hülse geneigt angeordnet und weiter vorzugsweise in einem Winkel von 31° bis 52°. Abhängig von dem Material des Bohrdrahts und der Bohrdrahthülse sowie von der Ausprägung der Innenkannte der ersten Durchbrechung (scharf oder ausgerundet) gleitet das vorangehende Ende des Bohrdrahts unterschiedlich gut an der Innenkante entlang. Bei einer Bohrdrahthülse aus Metall, beispielsweise aus Implantatstahl, Titan oder vergleichbaren Legierungen, ist der Winkelbereich von 28° bis 55° für ein sanftes entlang Gleiten des vorangehenden Endes des Bohrdrahts an der Innenkante vorteilhaft. Bei aufgerauten Bohrdrahtspitzen ist ein größerer Winkel vorzuziehen als bei glatt polierten Bohrdrahtspitzen.

Die Neigungswinkel der länglichen ersten Durchbrechungen sind in dieser Anmeldung angegeben als diejenigen Winkel, welche die Längsrichtung einer ersten länglichen Durchbrechung mit der Längsachse der Hülse in einer Abwicklung der Mantelfläche der Hülse einnehmen.

Der unrunde Querschnitt der ersten und zweiten Endkappe dient zum einen dazu, ein werkzeugfreies Lösen derselben von der Hülse zu ermöglichen, und andererseits verhindert solch ein Querschnitt, dass die Bohrdrahthülse rollen kann und damit auch, dass diese von dem Tisch rollen kann, auf der sie im OP angereichtet wird. Beispielsweise kann eine Endkappe die Außenform einer Flügelmutter haben oder eine eckige Form.

Gemäß einer vorteilhaften Ausgestaltung der vorliegenden Erfindung erstrecken sich die ersten Durchbrechungen in radialer Richtung der rohrförmigen, länglichen Hülse jeweils über 20% bis 60% des Außenumfangs der Hülse, vorzugsweise über 28% bis 47%. Dies bedeutet, dass sich die ersten Durchbrechungen in einem Winkelbereich von 72° bis 216° und vorzugsweise von ca. 100° bis 170° entlang des Umfangs der Hülse erstrecken. Mit diesen Abmessungen ist sicher gestellt, dass das vorangehende Ende des Bohrdrahts weit genug entlang der Innenkante der ersten Durchbrechung gleiten kann, um den Winkel $\beta$ so weit zu verringern, dass das vorangehende Ende des Bohrdrahts über die Innenkante der ersten Durchbrechung hinweg gleiten kann.

Prinzipiell versucht man, möglichst große und möglichst viele Spülöffnungen an einer Bohrdrahthülse vorzusehen, zumindest wenn sie auch als Reinigungs- und/oder Sterilisationslagerung konzipiert ist, also als eine Lagerung, in der der Bohrdraht auch während der Reinigung und/oder der Sterilisation verbleibt. Dies ermöglicht erforderlichenfalls eine gute Spülung des Innenraums der Bohrdrahthülse und somit eine gute Reinigung des Bohrdrahts bzw. der Bohrdrähte. Auch wird so gewährleistet, dass genug Sterilisationsmedium in alle Bereiche der Bohrdrahthülse gelangt, um die vollständige Sterilisation der Bohrdrähte in der Bohrdrahthülse sicher zu stellen. Auf der anderen Seite muss man so viele Stege zwischen den Spülöffnungen beibehalten, dass einerseits der Inhalt - hier die Bohrdrähte - nicht heraus fallen kann, und andererseits die Bohrdrahthülse noch hinreichend stabil ist. Dies hängt nicht zuletzt vom Material der Bohrdrahthülse ab.

Gemäß einer weiteren vorteilhaften Ausgestaltung der vorliegenden Erfindung sind die ersten Durchbrechungen in einem ersten Bereich der Mantelfläche der rohrförmigen, länglichen Hülse angeordnet. Der erste Bereich befindet sich dabei in einer Entfernung von etwa $a_1 = 2 * d_i$ bis $b_1 = 12 * d_i$ in axialer Richtung von einem Rand der Einführöffnung entfernt, vorzugsweise in einer Entfernung von etwa $a_2 = 2,5 * d_i$ bis $b_2 = 10 * d_i$. Dies bedeutet, dass der Winkel $\alpha$ zwischen der Einführrichtung des Bohrdrahts und der Längsachse der Hülse zwischen ca. $\pm 26,6°$ (entspricht $2 * d_i$) und ca. $\pm 4,7°$ (entspricht $12 * d_i$) liegen kann und vorzugsweise zwischen ca. $\pm 21,8°$ (entspricht $2,5 * d_i$) und ca. $\pm 5,7°$ (entspricht $10 * d_i$).

Der Zusammenhang zwischen Winkel $\alpha$ und der Lage des ersten Bereichs berechnet sich nach

$$\alpha = \tan^{-1}(d_i / x)$$

wobei x wahlweise $a_1$, $a_2$, $b_1$ oder $b_2$ ist.

Eine untere Grenze für den Winkel $\alpha$ für das Einführen des Bohrdrahts in die Bohrdrahthülse bedeutet dies aber nicht. Dies besagt lediglich, dass dann, wenn der Winkel $\alpha$ kleiner als ca. $\pm 4,7°$ bzw. vorzugsweise ca. $\pm 5,7°$ ist, die ersten Durchbrechungen nicht gegenüber der Längsachse der Hülse geneigt sein müssen, um das vorangehende Ende des Bohrdrahts über die Innenkanten der Durchbrechungen gleiten zu lassen. Die Werte $a_1$ bzw. $a_2$ bestimmen also die maximale Neigung der Einführrichtung des Bohrdrahts zu der Längsachse der Hülse. Die Werte $b_1$ bzw. $b_2$ bestimmen, wann der Winkel $\beta$ so klein geworden ist, dass das vorangehende Ende des Bohrdrahts über die Innenkannte der ersten Durchbrechung gleitet. In diesem ersten Bereich ist es besonders wichtig, dass die ersten Durchbrechungen vorgesehen sind.

Gemäß einer weiteren vorteilhaften Ausgestaltung der vorliegenden Erfindung ist eine Vielzahl von zweiten Durchbrechungen in einem zweiten Bereich der Mantelfläche der rohrförmigen, länglichen Hülse angeordnet sind. Dieser zweite Bereich befindet sich zwischen dem Rand der Einführöffnung und dem ersten Bereich und die zweiten Durchbrechungen sind im Wesentlichen kreisförmig ausgebildet und weisen eine kleinere Querschnittfläche als die ersten Durchbrechungen auf. Es ist vorteilhaft, wenn in diesem zweiten Bereich direkt in der Nähe der Einführöffnung der Bohrdrahthülse keine ersten Durchbrechungen vorgesehen sind, sondern zweite Durchbrechungen mit kleinerem Querschnitt. Dann kann der Bohrdraht, falls der Nutzer diesen in einem viel zu großen Winkel in die Bohrdrahthülse einführen möchte, dieser nicht durch eine der Durchbrechungen hindurch schieben, sodass der Bohrdraht dann doch aus der Bohrdrahthülse fällt. Da Insbesondere in der Nähe der Einführöffnung der Bohrdrahthülse für das auf und abschrauben der Endkappe eine erhöhte Steifigkeit erforderlich ist, sind im Wesentlichen kreisförmige Löcher mit relativ kleinem Querschnitt vorteilhaft.

Gemäß noch einer weiteren vorteilhaften Ausgestaltung der vorliegenden Erfindung weist die Mantelfläche der rohrförmigen, länglichen Hülse einen dritten Bereich auf, welcher nicht ein erster Bereich oder ein zweiter Bereich ist. In diesem dritten Bereich sind erste Durchbrechungen und/oder zweite Durchbrechungen und/oder andere Durchbrechungen vorgesehen. In diesem Bereich kommt es auf die genaue Form, Lage und Anordnung der Durchbrechungen nicht an, sodass hier beliebige Durchbrechungen vorgesehen werden können, solange diese als Spülöffnungen ausreichen und der Bohrdrahthülse eine hinreichende Steifigkeit belassen. In diesem dritten Bereich können auch Abschnitte vorgesehen sein, die keine Durchbrechungen aufweisen, um beispielsweise Beschriftungen vorzusehen.

Gemäß wieder einer vorteilhaften Ausgestaltung der vorliegenden Erfindung besitzt die erste axiale Endkappe und/oder die zweite axiale Endkappe einen polygonalen Außenquerschnitt und weiter vorzugsweise einen hexagonalen Außenquerschnitt. Ein polygonaler Querschnitt ist leicht herzustellen und ermöglicht ein einfaches Betätigen mit der Hand auch dann, wenn OP Handschuhe getragen werden. Insbesondere ein hexagonaler Außenquerschnitt, also ein Sechskant, ist besonders leicht herzustellen. Außerdem ist ein Sechskant noch so unrund, dass eine entsprechend ausgebildete Bohrdrahthülse nicht rollen kann und damit auch nicht vom Tisch fallen kann. Gleichzeitig sind bei einem Sechskant die Kanten nicht so spitz bzw. scharf, dass ein OP Handschuh verletzt wird. Bei einem Dreieckigen Außenquerschnitt kann dies leichter passieren.

Weitere Vorteile und Merkmale der Erfindung sind dem Fachmann aus den beigefügten Figuren und der detaillierten Beschreibung der Ausführungsbeispiele ersichtlich. Hierbei zeigt

Fig. 1     eine Ansicht eines ersten Ausführungsbeispiels der vorliegenden Erfindung;

Fig. 2     eine Ansicht der axialen Endabschnitte gemäß dem ersten Ausführungsbeispiel der Fig. 1;

Fig. 3     eine Ansicht eines axialen Endabschnitts gemäß dem ersten Ausführungsbeispiel der Fig. 1 mit einem Bohrdraht;

Fig. 4     eine Abwicklung einer Bohrdrahthülse gemäß einem zweiten Ausführungsbeispiel; und

Fig. 5     eine Abwicklung einer Bohrdrahthülse gemäß einem dritten Ausführungsbeispiel.

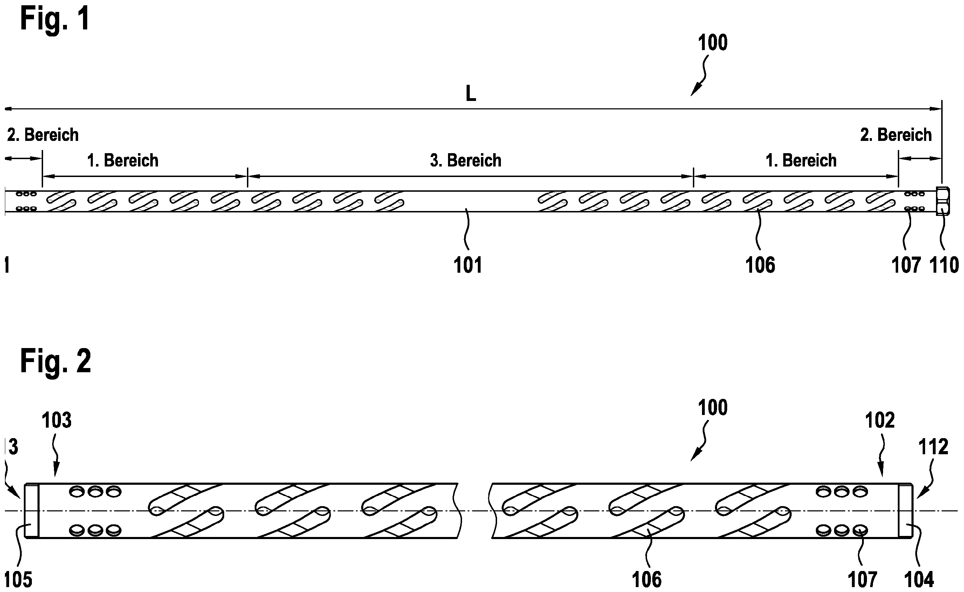

Ein erstes Ausführungsbeispiel der vorliegenden Erfindung ist unter Bezugnahme auf die Fig. 1 bis 3 im Detail beschrieben.

In Fig. 1 ist eine Bohrdrahthülse 100 gezeigt. Die Bohrdrahthülse 100 besteht aus einer rohrförmigen, länglichen Hülse 101 mit einer Länge L von 540mm, einem Innendurchmesser $d_i$ von 10mm und einem Außendurchmesser $d_a$ von 12m. Eine erste axiale Endkappe 110 und eine zweite axiale Endkappe 111 ist jeweils mit einem Innengewinde versehen. Ein erstes axiales Ende 102 und ein zweites axiales Ende 103 der Hülse 101 ist jeweils mit einem Außengewinde 104 bzw. 105 versehen. Die erste axiale Endkappe 110 und die zweite axiale Endkappe 111 sind mit ihrem Innengewinde werkzeugfrei auf den Gewinden 104, 105 aufschraubbar und von diesen abschraubbar und verschließen so werkzeugfrei lösbar die beiden axialen Enden 110, 111 der Hülse 101. Die Gewinde 104, 105 haben jeweils eine Länge von ca. 3 mm. Die axialen Enden 110, 111 können bei diesem Ausführungsbeispiel beide als Einführöffnungen 112, 113 für einen Bohrdraht 1 dienen. Selbstverständlich ist eine Einführöffnung 112, 113 in dieser Anmeldung auch immer eine Entnahmeöffnung, aus der ein Bohrdraht 1 aus der Bohrdrahthülse 100 entnommen werden kann. Die beiden Endkappen 110, 111 haben jeweils einen Sechskant als Außenquerschnitt, sodass diese auch mit einem entsprechenden Standardwerkzeug bedient werden können. Ein Sechskant als Außenquerschnitt der Endkappen 110, 111 ist auch deshalb besonders vorteilhaft, da ein Sechskant unrund genug ist, damit die Bohrdrahthülse 100 auch dann nicht ins Rollen gerät, wenn sie auf einer nicht ebenen Fläche abgelegt wird oder wenn sie versehentlich einen seitlichen Stoß abbekommt. Gleichzeitig sind die Winkel bei einem Sechskant nicht so spitz, dass sich ein Nutzer an einer scharfen Kante verletzen oder den Sterilhandschuh beschädigen könnte. Die Hülse 101 der Bohrdrahthülse 100 weist eine Vielzahl von länglichen ersten Durchbrechungen 106 auf. Diese ersten Durchbrechungen 106 sind insbesondere in den beiden ersten Bereichen, welche in Fig. 1 gezeigt sind, vorgesehen, aber auch teilweise in dem dritten Bereich. In den beiden zweiten Bereichen sind kreisförmige zweite Durchbrechungen 107 vorgesehen. Von den zweiten Durchbrechungen 107 sind jeweils 3 Stück gleichbeabstandet in axialer Richtung in jedem zweiten Bereich angeordnet, sodass sie eine Reihe von zweiten Durchbrechungen 107 bilden. Vier dieser Reihen sind gleichmäßig entlang des Umfangs der Hülse 101 angeordnet. Die zweiten Durchbrechungen weisen einen Durchmesser von ca. 3mm und sind in axialer Richtung ca. 1mm beabstandet. Die ersten Durchbrechungen 106 sind wie bereits beschrieben länglich ausgebildet und in einem Winkel von ca. 26° gegenüber der der Längsachse der rohrförmigen, länglichen Hülse 101 geneigt angeordnet. Die ersten Durchbrechungen haben jeweils eine Länge von ca. 20mm und eine Breite von ca. 3mm. Jeweils vier der schräg angeordneten ersten Durchbrechungen sind gleichmäßig entlang des Umfangs der Hülse 101 verteilt vorgesehen und bilden so einen Ring von ersten Durchbrechungen. In axialer Richtung der Hülse 101 sind mehrere dieser Ringe von ersten Durchbrechungen angeordnet. Zwischen zwei solchen Ringen von ersten Durchbrechungen ist ein Abstand von ca. 4mm vorgesehen. Die ersten Durchbrechungen erstrecken sich bei diesem Ausführungsbeispiel über etwas mehr als 45° der Umfangsrichtung der Hülse 101.

Die beiden ersten Bereiche beginnen jeweils in einer Entfernung von ca. $2,6 * d_i$ von den beiden axialen Enden 110, 111 der Hülse, also in einer Entfernung von ca. 26mm und erstrecken sich jeweils bis zu einer Entfernung von ca. $10 * d_i$ 100mm von den beiden axialen Enden 110, 111 der Hülse, also bis zu einer Entfernung von ca. 100mm. In dem ersten Bereich sind 5 Ringe von ersten Durchbrechungen vorgesehen. Außer den ersten Durchbrechungen 106 sind in dem ersten Bereich keine weiteren Durchbrechungen vorgesehen und in dem zweiten Bereich sind keine ersten Durchbrechungen 106 vorgesehen.

Zwischen den beiden ersten Bereichen der Hülse 101 ist ein dritter Bereich vorgesehen. In diesem Bereich sind weitere Ringe von ersten Durchbrechungen 106 vorgesehen, es ist dort aber auch ein Bereich vorgesehen, in dem keine Durchbrechungen vorhanden sind. In diesem Bereich können Beschriftungen vorgesehen sein wie beispielsweise Markenname, Artikelnummer oder zulassungs- oder nutzungsrelevante Informationen.

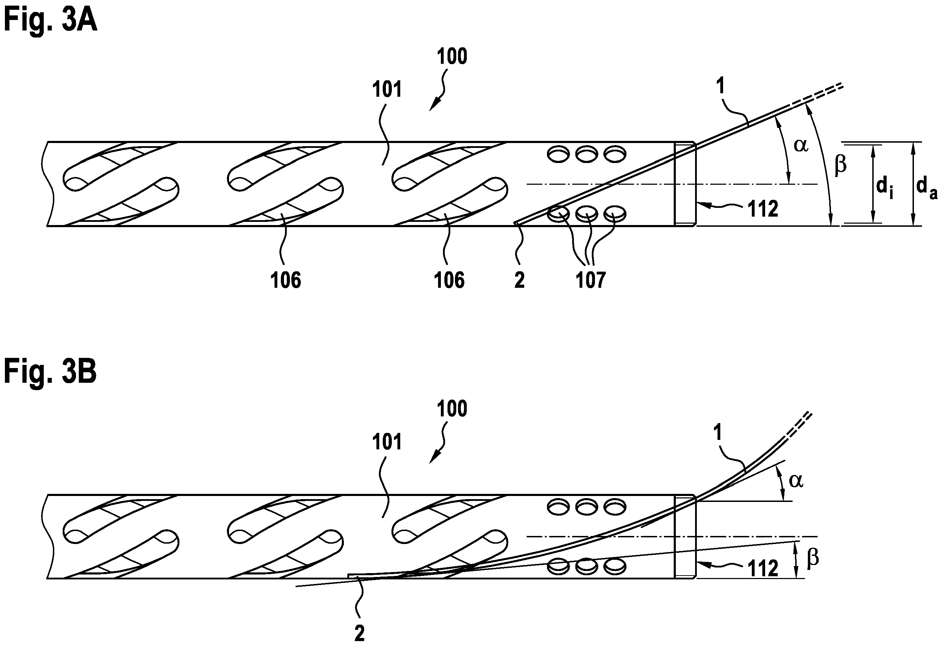

In Fig. 3A und 3B ist gezeigt, wie ein Bohrdraht 1 in die Einführöffnung 112 der Hülse 101 der Bohrdrahthülse 100 eingeführt wird. Der Bohrdraht wird in einem Winkel $\alpha$ eingeführt. Die Fig. 3A zeit ein extremes Beispiel, d.h. ein Einführen des Bohrdrahtes 1 in die Hülse 101 unter einem sehr großen Winkel $\alpha$, der in diesem Fall 22° beträgt. Das führende Ende 2 des Bohrdrahts 1 kommt in diesem Fall in dem Übergangsbereich zwischen dem zweiten Bereich und dem ersten Bereich der Hülse 101 in Anlage an die Innenwandfläche der Hülse 101. Der Winkel $\beta$ zwischen dem führenden Ende 2 des Bohrdrahts 1 und der Innenwandfläche der Hülse 101 beträgt zunächst $\beta = \alpha = 22°$. Wird der Bohrdraht 1 nun weiter in die Hülse 101 eingeschoben, gibt es im Wesentlichen zwei möglich Szenarien. Der Winkel $\alpha$ bleibt identisch und der Bohrdraht 100 wird beim weiteren Einführen gebogen oder der Bohrdraht 1 bleibt gerade und der Winkel $\alpha$ wird durch die Person, die den Bohrdraht 1 in die Hülse 101 einführt, verringert. In der Praxis passiert zumeist eine Kombination aus diesen beiden Szenarien, d.h. der Bohrdraht wird gebogen und gleichzeitig verringert der Nutzer den Winkel $\alpha$ ein wenig. In der Fig. 3B, welche eine Situation zeigt, in der der Bohrdraht 1 weiter in die Hülse 101 eingeschoben wurde, ist der Bohrdraht nun gebogen und so ist er Winkel $\beta$ zwischen dem führenden Ende 2 des Bohrdrahts und der Innenwandfläche der Hülse 101 nun kleiner als der Einführwinkel $\alpha$ des Bohrdrahts in die Einführöffnung 112 der Hülse 101.

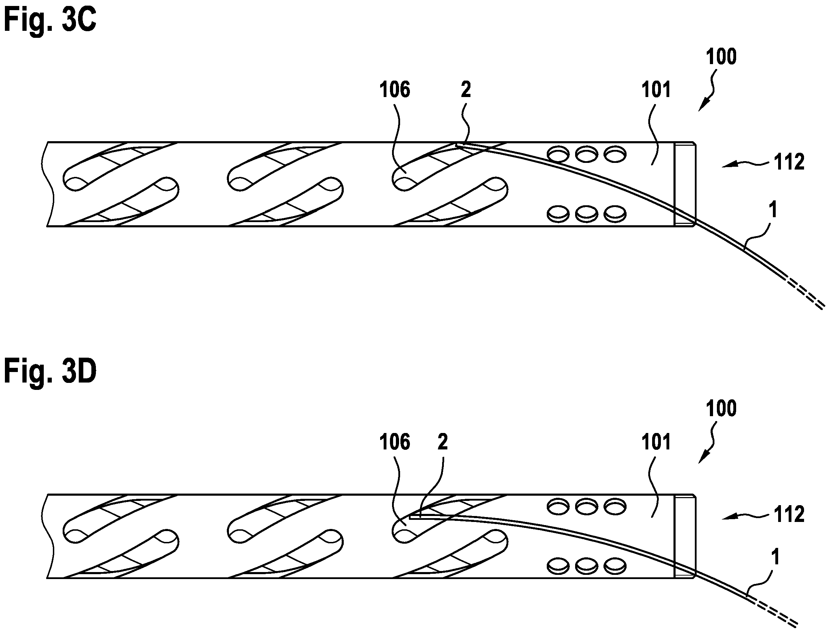

Taucht das führende Ende 2 des Bohrdrahts 1 bei diesem Vorgang nun in ein erste Durchbrechung 106 ein, wie dies in Fig. 3C gezeigt ist, stößt das führende Ende 2 an eine Längskante dieser Durchbrechung 106 und gleitet an dieser entlang, da diese gegenüber der Längsachse der Hülse 101 geneigt ist. Dadurch wird das führende Ende 2 des Bohrdrahts 1 anteilig in einer Umfangsrichtung des Hülse 101 bewegt. Wie dies in Fig. 3D gezeigt ist, verringert dieser Effekt den Winkel $\beta$ zusätzlich deutlich und sorgt dafür, dass dieser Winkel $\beta$ so klein wird, dass das führende Ende 2 des Bohrdrahts 1 über diese Längskante hinweg gleitet. Von der Position in Fig. 3C zu der in Fig. 3D wird das führende Ende 2 des Bohrdrahts 1 also in der Ansicht dieser Figuren nicht nur weiter nach links bewegt, sondern auch in einer Richtung auf den Betrachter zu. Es entsteht also ein Effekt in einer dritten Dimension senkrecht zur Darstellungsebene der Fig. 3C und 3D.

Die Hülse dieses Ausführungsbeispiels ist aus Edelstahl (1.4301) hergestellt. Die ersten und zweiten Durchbrechungen 106, 107 wurden mittels Laserschneiden hergestellt.

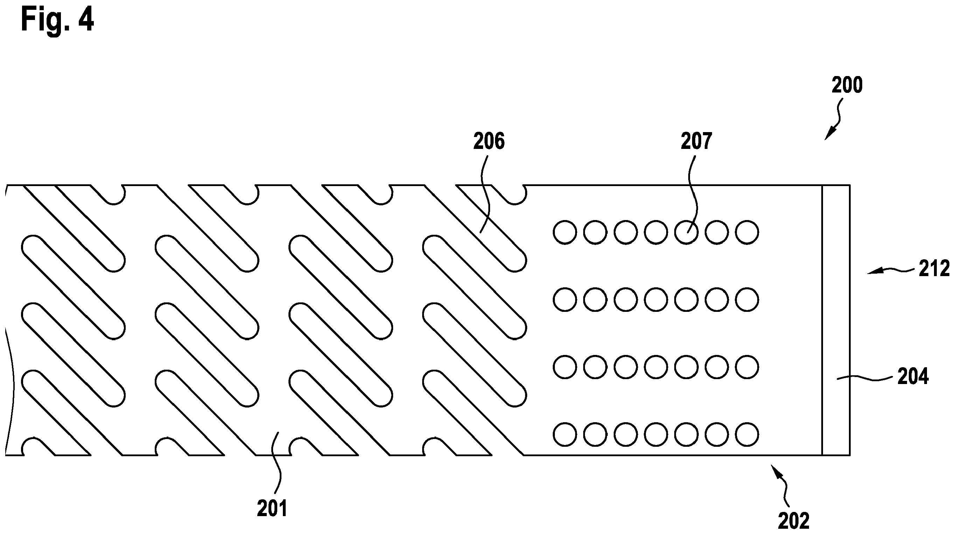

In der Fig. 4 ist eine Abwicklung eines Endabschnitts einer Hülse 201 einer Bohrdrahthülse 200 eines zweiten Ausführungsbeispiels gezeigt. Der prinzipielle Aufbau dieses zweiten Ausführungsbeispiels ist zu dem des ersten Ausführungsbeispiels gleich. Im Folgenden werden nur die wesentlichen Unterschiede beschrieben.

Ein erstes axiales Ende 202 der Hülse 201 weist einen Rand einer Einführöffnung 212 auf, welche mit einem Außengewinde 204 versehen ist, um mit einer ersten Endkappe, welche ein dem Außengewinde 204 entsprechendes Innengewinde aufweist, werkzeugfrei lösbar verschlossen zu werden. Das zweite axiale Ende ist mit einer zweiten Endkappe fest verschlossen. Dadurch ist bei dieser Bohrdrahthülse nur jeweils ein erster Bereich, ein zweiter Bereich und ein dritter Bereich ausgebildet. Die erste Endkappe weist eine hexagonale Außenkontur auf, die zweite Endkappe eine quadratische Außenkontur. Das Außengewinde hat eine Länge von ca. 5mm

Die ersten Durchbrechungen 206 dieses Ausführungsbeispiels sind ca. 20mm lang, 3mm breit und um ca. 51,5° gegenüber der Längsachse der Hülse 201 geneigt. Die zweiten Durchbrechungen 207 sind kreisrund und weisen einen Durchmesser von ca. 3mm auf. Es sind jeweils 7 dieser zweiten Durchbrechungen 207 in axialer Richtung der Hülse 201 in dem zweiten Bereich angeordnet. Der erste Bereich erstreckt sich von 42mm Entfernung zur Einführöffnung 212 bis 120mm Entfernung zur Einführöffnung 212 bei einem Innendurchmesser $d_i$ der Hülse von 10mm.

Die Bohrdrahthülse 201 dieses Ausführungsbeispiels ist aus chirurgischem Stahl gefertigt. Die ersten und zweiten Durchbrechungen 206, 207 sind mittels Laserschneiden hergestellt.

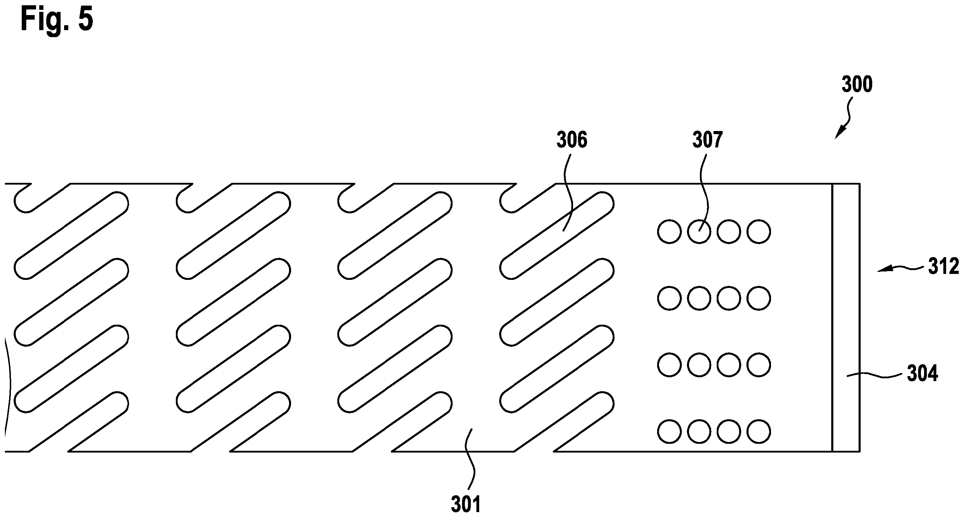

Ein drittes Ausführungsbeispiel der vorliegenden Erfindung ist in der Fig. 5 gezeigt. Es sind im Folgenden wieder nur die Unterschiede zum ersten Ausführungsbeispiel beschrieben.

Die beiden ersten Bereiche erstrecken sich von 20mm Entfernung zum Rand der Einführöffnung 312 bis 90mm Entfernung zum Rand der Einführöffnung 312 bei einem Innendurchmesser $d_i$ der Hülse 301 von ebenfalls 10mm auf. Das Außengewinde 304 hat eine Länge von ca. 4mm. Die ersten Durchbrechungen 306 haben eine Länge von ca. 18,5 mm, eine Breite von ca. 3mm und sind um ca. 31,5° gegenüber der Längsachse der Hülse 301 geneigt. Jeweils vier zweite Durchbrechungen 307 sind in axialer Richtung der Hülse 301 vorgesehen. Der Außendurchmesser $d_a$ der Hülse 301 beträgt 11,5mm, sodass die Wandstärke der Hülse 301 geringer ist als bei dem ersten Ausführungsbeispiel.

Dem Fachmann ist klar, dass über die konkreten Ausführungsbeispiele hinaus zahlreiche Ausführungsformen der vorliegenden Erfindung möglich sind. Beispielsweise können die ersten Durchbrechungen in allen Ausführungsbeispielen gebogen oder geschwungen sein, eine veränderliche Breite aufweisen und dergleichen. Der Innendurchmesser kann für dickere Führungsdrähte oder für die Aufnahme einer größeren Anzahl von Führungsdrähten größer gewählt werden. Es kommen verschiedenste Materialien zur Herstellung der Bohrdrahthülsen in Frage, beispielsweise Kobalt-Chrom Legierungen sowie alle weiteren Metalle und Legierungen, welche im chirurgischen Bereich eingesetzt werden und resterilisierbar sind.

Die zweiten Durchbrechungen können oval oder polygonal ausgebildet werden und es können mehr oder weniger als vier dieser zweiten Durchbrechungen in Umfangsrichtung der Hülse vorgesehen sein. Die ersten Durchbrechungen müssen nicht in ringartigen Strukturen vorgesehen sein sondern können zueinander versetzt angeordnet sein. Auch von den ersten Durchbrechungen können mehr oder weniger als vier in Umfangsrichtung der Hülse verteilt vorgesehen sein. Es ist auch möglich, die ersten und zweiten Durchbrechungen nicht gleichmäßig in axialer Richtung und/oder Umfangsrichtung der Hülse vorzusehen. Zudem können einzelne Merkmale der verschiedenen Ausführungsbeispiele geeignet miteinander kombiniert oder vertauscht werden.

**Bezugszeichenliste**

| | |
|---|---|
| $\alpha$ | Schwenkbereich |
| $d_i$ | Innendurchmesser |
| $d_a$ | Außendurchmesser |
| L | Länge |
| 1 | Bohrdraht |
| 2 | führendes Ende |
| 100, 200, 300 | Bohrdrahthülse |
| 101, 201, 301 | Hülse |
| 102, 202 | erstes axiales Ende |
| 103 | zweites axiales Ende |
| 104, 105, 204, 304 | Außengewinde |
| 106, 206, 306 | erste Durchbrechungen |
| 107, 207, 307 | zweite Durchbrechungen |
| 110 | erste axiale Endkappe |
| 111 | zweite axiale Endkappe |
| 112, 212, 312 | Entnahmeöffnung |
| 113 | Entnahmeöffnung |

**Patentansprüche**

**1.** Bohrdrahthülse (100, 200, 300) bestehend aus einer rohrförmigen, länglichen Hülse (101, 201, 301) mit einer Länge L, einem Innendurchmesser $d_i$ und einem Außendurchmesser $d_a$,

mit einer ersten axialen Endkappe (110) und einer zweiten axialen Endkappe (111), wobei die erste axiale Endkappe und/oder die zweite axiale Endkappe mit einem Gewinde werkzeugfrei lösbar an der rohrförmigen, länglichen Hülse befestigbar ist und eine Einführöffnung (112, 212, 312) der rohrförmigen, länglichen Hülse lösbar verschließt,

die erste axiale Endkappe und/oder die zweite axiale Endkappe einen nicht rotationssymmetrischen Außenquerschnitt aufweist, und

die rohrförmige, längliche Hülse in ihrer Mantelfläche eine Vielzahl von ersten Durchbrechungen (106, 206, 306) aufweist, **dadurch gekennzeichnet, dass**

die ersten Durchbrechungen länglich ausgebildet sind und in einem Winkel von 25° bis 65°gegenüber der der Längsachse der rohrförmigen, länglichen Hülse geneigt sind, vorzugsweise in einem Winkel von 28° bis 55° und weiter vorzugsweise in einem Winkel von 31° bis 52°.

2. Bohrdrahthülse gemäß Anspruch 1, wobei sich die ersten Durchbrechungen in radialer Richtung der rohrförmigen, länglichen Hülse jeweils über 20% bis 60% des Außenumfangs der Hülse erstrecken und vorzugsweise über 28% bis 47%.

3. Bohrdrahthülse gemäß einem der vorangehenden Ansprüche, wobei die ersten Durchbrechungen in einem ersten Bereich der Mantelfläche der rohrförmigen, länglichen Hülse angeordnet sind, und

sich der erste Bereich in einem Abschnitt befindet, welcher sich von $2 * d_i$ bis $12 * d_i$ in axialer Richtung von einem Rand der Einführöffnung entfernt befindet, und vorzugsweise in einem Abschnitt, welcher sich von $2,5 * d_i$ bis $10 * d_i$ in axialer Richtung von einem Rand der Einführöffnung entfernt befindet.

4. Bohrdrahthülse gemäß Anspruch 3, wobei eine Vielzahl von zweiten Durchbrechungen (107, 207, 307) in einem zweiten Bereich der Mantelfläche der rohrförmigen, länglichen Hülse angeordnet sind, und

sich der zweite Bereich zwischen dem Rand der Einführöffnung und dem ersten Bereich befindet, wobei die zweiten Durchbrechungen im Wesentlichen kreisförmig ausgebildet sind und eine kleinere Querschnittfläche als die ersten Durchbrechungen aufweisen.

5. Bohrdrahthülse gemäß Anspruch 4, wobei die Mantelfläche der rohrförmigen, länglichen Hülse in einem dritten Bereich, welcher nicht ein erster Bereich oder ein zweiter Bereich ist, erste Durchbrechungen und/oder zweite Durchbrechungen und/oder andere Durchbrechungen aufweist.

6. Bohrdrahthülse gemäß einem der vorangehenden Ansprüche, wobei die erste axiale Endkappe und/oder die zweite axiale Endkappe einen polygonalen Außenquerschnitt und weiter vorzugsweise einen hexagonalen Außenquerschnitt besitzt.


**Claims**

1. Drill wire sleeve (100, 200, 300) comprising a tubular, elongate sleeve (101, 201, 301) having a length L, an inside diameter $d_i$ and an outside diameter $d_a$, with a first axial end cap (110) and a second axial end cap (111), wherein the first axial end cap and/or the second axial end cap can be releasably fastened to the tubular, elongate sleeve in a tool-free manner by a thread and releasably closes an insertion opening (112, 212, 312) of the tubular, elongate sleeve, the first axial end cap and/or the second axial end cap have/has a non-rotationally symmetrical external cross section, and the tubular, elongate sleeve has a plurality of first apertures (106, 206, 306) in its lateral surface, **characterized in that** the first apertures are elongate in form and are inclined at an angle of 25° to 65° with respect to the longitudinal axis of the tubular, elongate sleeve, preferably at an angle of 28° to 55° and further preferably at an angle of 31° to 52°.

2. Drill wire sleeve according to Claim 1, wherein the first apertures extend in the radial direction of the tubular, elongate sleeve in each case over 20% to 60% of the outer circumference of the sleeve and preferably over 28% to 47%.

3. Drill wire sleeve according to either of the preceding claims, wherein the first apertures are arranged in a first region of the lateral surface of the tubular, elongate sleeve, and the first region is situated in a portion which is situated at a distance of $2 * d_i$ to $12 * d_i$ in the axial direction from an edge of the insertion opening, and preferably in a portion which is situated at a distance of $2.5 * d_i$ to $10 * d_i$ in the axial direction from an edge of the insertion opening.

4. Drill wire sleeve according to Claim 3, wherein a plurality of second apertures (107, 207, 307) are arranged in a second region of the lateral surface of the tubular, elongate sleeve, and the second region is situated between the edge of the insertion opening and the first region, wherein the second apertures are substantially circular in form and have a smaller cross-sectional area than the first apertures.

5. Drill wire sleeve according to Claim 4, wherein the lateral surface of the tubular, elongate sleeve has first apertures and/or second apertures and/or other apertures in a third region which is not a first region or a second region.

6. Drill wire sleeve according to one of the preceding claims, wherein the first axial end cap and/or the second axial end cap have/has a polygonal external cross section and further preferably a hexagonal external cross section.

**Revendications**

1. Gaine de broche de Kirschner (100, 200, 300), constituée d'une gaine allongée tubulaire (101, 201, 301) de longueur L, de diamètre intérieur $d_i$ et de diamètre extérieur $d_a$, avec un premier embout axial (110) et un deuxième embout axial (111), le premier embout axial et/ou le deuxième embout axial pouvant être fixés de manière amovible sans outil au moyen d'un filetage à la gaine allongée tubulaire et fermant de manière amovible une ouverture d'introduction (112, 212, 312) de la gaine allongée tubulaire, le premier embout axial et/ou le deuxième embout axial présentant une section transversale extérieure asymétrique en rotation, et la gaine allongée tubulaire présentant dans sa surface d'enveloppe une pluralité de premiers orifices (106, 206, 306), **caractérisée en ce que** les premiers orifices sont réalisés sous forme allongée et sont inclinés suivant un angle de 25° à 65° par rapport à l'axe longitudinal de la gaine allongée tubulaire, de préférence suivant un angle de 28° à 55° et plus préférablement suivant un angle de 31° à 52°.

2. Gaine de broche de Kirschner selon la revendication 1, dans laquelle les premiers orifices s'étendent dans la direction radiale de la gaine allongée tubulaire à chaque fois sur 20 % à 60 % de la périphérie extérieure de la gaine, de préférence sur 28 % à 47 %.

3. Gaine de broche de Kirschner selon l'une quelconque des revendications précédentes, dans laquelle les premiers orifices sont disposés dans une première région de la surface d'enveloppe de la gaine allongée tubulaire et la première région se trouve dans une portion qui est éloignée d'un bord de l'ouverture d'introduction dans la direction axiale de $2*d_i$ à $12*d_i$, et de préférence dans une portion qui est éloignée d'un bord de l'ouverture d'introduction dans la direction axiale de $2,5*d_i$ à $10*d_i$.

4. Gaine de broche de Kirschner selon la revendication 3, dans laquelle une pluralité de deuxièmes orifices (107, 207, 307) sont disposés dans une deuxième région de la surface d'enveloppe de la gaine allongée tubulaire et la deuxième région se trouve entre le bord de l'ouverture d'introduction et la première région, les deuxièmes orifices étant réalisés essentiellement sous forme circulaire et présentant une plus petite surface en section transversale que les premiers orifices.

5. Gaine de broche de Kirschner selon la revendication 4, dans laquelle la surface d'enveloppe de la gaine allongée tubulaire présente des premiers orifices et/ou des deuxièmes orifices et/ou d'autres orifices dans une troisième région qui n'est ni une première région ni une deuxième région.

6. Gaine de broche de Kirschner selon l'une quelconque des revendications précédentes, dans laquelle le premier embout axial et/ou le deuxième embout axial possèdent une section transversale extérieure polygonale et en outre de préférence une section transversale extérieure hexagonale.

# Fig. 1

# Fig. 2

## Fig. 3A

## Fig. 3B

EP 3 468 499 B1

Fig. 3C

Fig. 3D

Fig. 4

200

212

204

202

207

206

201

EP 3 468 499 B1

**Fig. 5**

**EP 3 468 499 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- CN 205359661 U
- US 20170065794 A1
- DE 3119173 A1